# EUROPÄISCHE PATENTSCHRIFT

(11) **EP 2 012 869 B1**
(45) Veröffentlichungstag und Bekanntmachung des Hinweises auf die Patenterteilung: **17.04.2013**
(21) Anmeldenummer: 07722321.2
(22) Anmeldetag: 27.04.2007
(51) Int. Cl.: A61N 1/05, A61N 1/32, A61B 17/58

(54) **STIMULATIONSVORRICHTUNG FÜR DIE OSTEOSYNTHESE UND DIE ENDOPROTHETIK**
STIMULATION DEVICE FOR OSTEOSYNTHESIS AND ENDOPROSTHETICS
DISPOSITIF DE STIMULATION POUR OSTÉOSYNTHÈSE ET ENDOPROTHÈSE

(30) Priorität: 01.05.2006 DE 102006019955; 17.07.2006 DE 102006032957
(43) Veröffentlichungstag der Anmeldung: 14.01.2009
(73) Patentinhaber: Neue Magnetodyn GmbH, 80333 München (DE)
(72) Erfinder: KRAUS, Werner, 80539 München (DE); KRAUS, Stephanie, 80538 München (DE); STEPHAN, Heribert, 80689 München (DE)
(74) Vertreter: Schumacher & Willsau
(86) Internationale Anmeldenummer: PCT/DE2007/000764
(87) Internationale Veröffentlichungsnummer: WO 2007/124731

(56) Entgegenhaltungen:
- EP-A2- 0 781 532
- US-A- 4 306 564
- US-A- 5 292 252
- US-A- 5 397 342
- US-A1- 2004 176 829
- US-B1- 6 778 861

## Beschreibung

Die Erfindung betrifft eine Stimulationsvorrichtung zur Implantation in den menschlichen Körper, mit einer Spulenanordnung, einer mit einem ersten Pol der Spulenanordnung verbundenen ersten Elektrode und einer mit einem zweiten Pol der Spulenanordnung verbundenen zweiten Elektrode.

Derartige Stimulationsvorrichtungen sind auf dem Gebiet der Osteosynthese sowie der Endoprothetik bekannt. Eine Stimulationsvorrichtung mit den Merkmalen des Oberbegriffs des Anspruchs 1 ist aus US-B-6 778 861 bekannt.

Die Osteosynthese dient der belastungsstabilen Fixation der Fragmente eines gebrochenen oder kranken Knochens in seiner unverletzten, natürlichen Form durch implantierte Schrauben, Stützplatten, Drähte, Knochenmarknägel und dergleichen, die im Allgemeinen aus nicht rostenden Stahl- oder Titanlegierungen gefertigt sind. Diese Osteosynthesemittel ermöglichen die rasche Mobilisierung des Patienten bei gleichzeitiger Ruhigstellung des lädierten Knochens, die eine unerlässliche Voraussetzung für seine Heilung ist.

Die Endoprothetik dient der Implatation von Prothesen, insbesondere Gelenkprothesen, beispielsweise in der Hüfte.

Die Zahl der Patienten mit knochen- und gelenktragenden sowie stützenden Metallimplantaten im Skelett ist in den zurückliegenden zwei Jahrzehnten exponentiell angestiegen. Die Gründe hierfür bilden die Zunahme der unfallbedingten komplizierten Knochenfrakturen und ganz besonders auch der degenerativen Erkrankungen der Gelenke (Arthrosen, Nekrosen), die in immer früherem Lebensalter zum künstlichen Gelenkersatz durch eine Endoprothese führen. Mit der Zunahme des Altersdurchschnitts der Menschen um nahezu zehn Jahre - während der zurückliegenden fünf Jahrzehnte - wächst auch der Anspruch auf die beschwerdefreie Funktionsdauer eines Kunstgelenks. Wurde dieser im sechsten bis siebten Jahrzehnt des vergangenen Jahrhunderts mit 15 bis 20 Jahren erfüllt, so steht die Technik heute vor der Aufgabe, eine möglichst verlustfreie Mobilität des Trägers eines Kunstgelenks über bis zu drei Jahrzehnte oder länger zu gewährleisten. Diesem wachsenden Anspruch an die biomechanische Toleranz des biologischen Lagers eines Langzeitimplantates im Knochenskelett versucht man durch verträglichere Materialien, wie Titanlegierungen und patientenindividuelle Formgebungen unter größtmöglicher Schonung der versorgenden Blutgefäße zu entsprechen.

Trotz der beachtlichen Fortschritte in der Anpassung der Fremdkörperimplantate an die individualbiologischen und - physiologischen Gegebenheiten entstehen mit dem wachsenden Anspruch der Patienten an die Mobilität und die Funktionsdauer des Implantates neue Probleme, die einer stimulierenden Vermittlung zwischen dem Fremdkörper und seinem biologischen Lager bedürfen. Dass diese Aufgabe durch die Applikation extrem niederfrequenter elektromagnetischer Wechselfelder mit einer Frequenz von 3 bis 30 Hz bei reiner Sinusform (Oberwellenanteil < 1 %) in Verbindung mit einer implantierten Spule (Sekundärinduktivität dem sog. Übertrager) im elektrischen Kontakt mit den Metallbestandteiten der Osteosynthese und der Gelenkendoprothetik auch in Fällen der extremen Knochenheilungsstörung zu lösen ist, wurde in zahlreichen Grundlagenexperimenten und klinischen Studien des Antragsstellers innerhalb von dreieinhalb Jahrzehnten nachgewiesen und publiziert. Die Mehrzahl der Patienten mit Stütz- bzw. Gelenkimplantaten war mit Keimen infiziert, die heute als biologisch multiresistent bezeichnet werden (MRSA = Multiresistenter Staphylococcus Aureus) und ein wachsendes Problem in der orthopädischen und unfallchirurgischen Klinik darstellen. Offenbar sind Keime, die sich an Langzeitimplantaten in Form von "Biofilmen" ansiedeln und sich durch Schleimhüllen schützen, für Antibiotika nicht mehr erreichbar. Die Adhärenz von Keimfilmen an Metallimplantaten kann mit der elektrischen Aktivierung Ihrer Oberfläche durch die verfahrensgemäße elektromagnetische Induktion offenbar vermieden werden.

Die Technik der Übertragung funktioniert nach dem Transformatorprinzip: Die verletzte oder kranke Körperregion wird von einem extrem niederfrequenten sinusförmig verlaufenden Magnetfeld mit einer Frequenz von ca. 1 bis 100 Hz - vorzugsweise von 3 bis 30 Hz - und einer magnetischen Flussdichte von 0,5 bis 5 mT (5 bis 50 Gauß) durchflutet, das durch einen Funktionsstromgenerator in einer oder mehreren - primären - äußeren Stromspulen erzeugt wird, in die das mit den Osteosynthesemitteln oder der Endoprothese versehene Körperteil eingebracht wird. Diese extrem niederfrequenten elektromagnetischen Felder durchdringen weitgehend verlustfrei das Gewebe, einschließlich eventuelle Kleidung und einen Gipsverband, sowie die unmagnetischen (austenitischen) Stützmetalle der Osteosynthese oder der Endoprothetik. Im elektrischen Kontakt mit diesen wird eine - sekundäre - Spulenanordnung, der so genannte Übertrager, implantiert. Die in dem Übertrager induzierten Elektropotentiale werden so im Bereich der Knochenläsion sowie allgemein in dem an die Osteosynthesemittel beziehungsweise die Endoprothese angrenzenden Gewebe zur Wirkung gebracht. Als Behandlungsparameter dienen elektrische Spannung, Frequenz, Intensität, Signalform und die Behandlungszeit, die durch die indikationsspezifische Programmierung des das induzierende Magnetfeld bestimmenden Funktionsstrom-Generators bestimmt werden.

Grundsätzlich stehen somit Techniken zur Verfügung, um die Gefahren der Osteosynthese sowie der Endoprothetik zu vermindern.

Problematisch ist jedoch insbesondere die Situation, in der eine Endoprothese beziehungsweise ein Osteosynthesemittel ohne Befähigung zur Anwendung der beschriebenen, elektromagnetische Wechselfelder nutzenden Therapie bereits seit Langem implantiert ist und ein Wechsel des Stütz- bzw. Gelenkimplantates im heilungsresistenten, infizierten Knochen ein für den Chirurgen nicht mehr kalkulierbares Risiko bedeutet. Insbesondere für viele zumeist ältere Patienten mit infektionsgefährdeten Stütz- und Gelenkimplantaten ist die komplizierte Operation zum Implantatwechsel mit einem deutlich erhöhten Überlebensrisiko verbunden.

Der Erfindung liegt die Aufgabe zugrunde, eine Technik zur Vermeidung der Notwendigkeit eines Implantatwechsels zur Verfügung zu stellen, insbesondere bei Risikopatienten.

Diese Aufgabe wird mit den Merkmalen des unabhängigen Anspruchs gelöst.

Vorteilhafte Ausführungsformen der Erfindung sind in den abhängigen Ansprüchen angegeben.

Die Erfindung baut auf der gattungsgemäßen Stimulationsvorrichtung dadurch auf, dass die zweite Elektrode als elastisches Kontaktelement ausgebildet ist. Hierdurch wird es ermöglicht, im Knochenbereich implantierte Metallteile über das elastische Kontaktelement elektrisch zu kontaktieren. Auf diese Weise wird das bereits implantierte Metallteil zur Elektrode, während der elektrisch mit der Spulenanordnung verbundene Teil der Stimulationsvorrichtung die Gegenelektrode hierzu bildet. Folglich kann ohne Austausch des Implantats dieses in die eingangs beschriebene Therapie unter Verwendung niederfrequenter elektromagnetischer Wechselfelder einbezogen werden.

Nützlicherweise ist vorgesehen, dass die Stimulationsvorrichtung einen eine Achse definierenden Schaft aufweist, die Spulenanordnung in einem radial innen liegenden Aufnahmenbereich des Schaftes angeordnet ist und zumindest ein Teil des Schaftes die erste Elektrode bildet. Die Stimulationsvorrichtung ist somit als längliches Element ausgebildet, wodurch sie sich zur Einführung in kleine Öffnungen des Körpers und insbesondere des Knochens eignet. Die Spulenanordnung kann flüssigkeits- und gasdicht innerhalb des Schaftes der Stimulationsvorrichtung sicher untergebracht sein.

Die Erfindung ist in vorteilhafter Weise dadurch weitergebildet, dass an einem Endbereich des Schaftes ein elektrisch isolierendes Endstück ansetzt, durch welches eine elektrische Verbindung zu dem an der dem Schaft abgewandten Seite des Endstücks angeordneten elastischen Kontaktelement geführt ist. Das elektrisch isolierende Endstück dient dazu, das elastische Kontaktelement vom Rest des elektrisch leitenden Vorrichtungskörpers zu isolieren, und es ermöglicht weiterhin die Durchführung der elektrischen Verbindung von der im Schaft angeordneten Spulenanordnung zu dem außen liegenden Kontaktelement.

Es kann vorgesehen sein, dass das Kontaktelement in dem Endstück fixiert ist. Beispielsweise kann das Kontaktelement eingesintert oder mit Epoxydharz eingeklebt sein; zusätzliche Befestigungsmittel sind somit nicht erforderlich.

Gemäß einer Variante der vorliegenden Erfindung ist vorgesehen, dass das Kontaktelement zumindest teilweise aus federhartem Stahl besteht.

Ebenfalls kann vorgesehen sein, dass das Kontaktelement zumindest teilweise aus federhartem Titan besteht.

Zur Herstellung eines guten elektrischen Kontaktes zwischen dem Kontaktelement und dem bereits implantierten Metallteil ist nützlicherweise vorgesehen, dass das Kontaktelement mindestens einen gewellten Draht aufweist.

Ebenfalls kann die Erfindung so ausgelegt sein, dass das Kontaktelement mindestens einen wendelförmigen Draht aufweist.

Es ist bevorzugt, dass die Stimulationsvorrichtung als Knochenschraube ausgebildet ist, die ein Außengewinde aufweist. Eine Knochenschraube lässt sich vorteilhaft zum Einsatz bringen, da diese in Knochen sicher fixiert werden kann, so dass sich auch die Relativposition der Stimulationsvorrichtung gegenüber dem bereits implantierten Metallteil nicht oder nur unwesentlich ändert. Des Weiteren muss zur Fixierung der Knochenschraube kein weiteres Hilfsmittel implantiert werden. Auch wenn die Auslegung der Stimulationsvorrichtung als Knochenschraube bevorzugt sein mag, ist festzuhalten, dass jegliche andere Formen denkbar sind. Mitunter ist zur Fixierung von Stimulationsvorrichtungen anderer Form die Implantation einer zusätzlichen Fixiervorrichtung erforderlich.

Die Erfindung ist ferner dadurch besonders nützlich weitergebildet, dass die äußere Oberfläche der Stimulationsvorrichtung zumindest teilweise mit einer die Oberfläche der Stimulationsvorrichtung vergrößernden und die Anlagerung von Bakterien vermeidenden, elektrisch leitfähigen Beschichtung versehen ist. Bakterizide Beschichtungen sind bekannt. Wählt man eine elektrisch leitfähige bakterizide Beschichtung, die die Oberfläche der Stimulationsvorrichtung vergrößert, so kommt es zu einer Verstärkung des bakteriziden Effektes, nämlich aufgrund der vergrößerten Oberfläche zur Übertragung des elektrischen Feldes auf das umgebende Gewebe.

In diesem Zusammenhang ist bevorzugt, dass die Beschichtung Silber aufweist. Eine Silberbeschichtung kann beispielsweise direkt auf Implantate aus Stahl- oder Titanlegierungen mittels einer Sputtertechnik aufgebracht werden.

Nützlicherweise kann aber auch vorgesehen sein, dass zwischen der Oberfläche der Vorrichtung und der Beschichtung eine poröse Zwischenschicht vorgesehen ist. Die elektrisch leitfähige Verbindung der Beschichtung mit der unter der Zwischenschicht liegenden Oberfläche der Stimulationsvorrichtung wird durch die umgebende Körperflüssigkeit und/oder durch direkten Kontakt der Silberpartikel mit der Oberfläche zur Verfügung gestellt. Die poröse Zwischenschicht besteht beispielsweise aus Keramik oder Kunststoff. Der Erfindung liegt die Erkenntnis zugrunde, dass eine Stimulationsvorrichtung, insbesondere eine Knochenschraube, mit integrierter Sekundär-Induktions-Spule und einer zungenförmigen Elektrode an der Spitze der Vorrichtung durch einen chirurgisch-minimalinvasiven Eingriff in permanent leitenden Kontakt mit der Oberfläche eines Metall-Stütz- bzw. Gelenkimplantates gebracht werden kann. Durch die Induktion der Sekundärspule durch ein äußeres Elektromagnetfeld wird die Oberfläche des Langzeitimplantates zur Elektrode mit einer elektrischen Potentialdifferenz zum Schaft der Stimulationsvorrichtung von 500 bis 700 mV. Durch diese Anordnung werden insbesondere die folgenden Wirkungen erzielt:
1. Die Ansiedlung von Keimen wird vermieden.
2. Die Multiresistenz gegen Antibiotika wird aufgehoben.
3. Der Knochen wächst an das Langzeitimplantat heran und verleiht ihm wieder festen Sitz.

Die Erfindung wird nun mit Bezug auf die begleitenden Zeichnungen anhand bevorzugter Ausführungsformen beispielhaft erläutert.

Es zeigen:
- Figur 1: eine Schnittdarstellung einer erfindungsgemäßen Stimulationsvorrichtung;
- Figur 2: eine schematische Darstellung einer in einen Oberschenkelknochen eingebrachten Stimulationsvorrichtung zur Kontaktierung einer Hüftkopfkappenprothese;
- Figur 3: eine schematische Darstellung von zwei in den Oberschenkelknochen eingeschraubten Stimulationsvorrichtungen zur Kontaktierung des Schaftes einer Hüftgelenkprothese;
- Figur 4: eine schematische Darstellung einer in einen Röhrenknochen eingebrachten Stimulationsvorrichtung zur Kontaktierung eines Marknagels;
- Figur 5: eine schematische Darstellung einer in einen gebrochenen Knochen eingebrachten Stimulationsvorrichtung zur Kontaktierung einer Stützplatte und

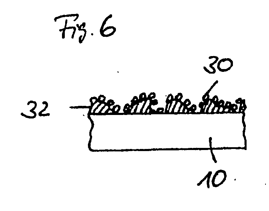

- Figur 6: einen Schnitt durch die Oberfläche einer erfindungsgemäßen Stimulationsvorrichtung mit einer die Oberfläche vergrößernden Beschichtung.

Bei der nachfolgenden Beschreibung der bevorzugten Ausführungsformen der vorliegenden Erfindung bezeichnen gleiche Bezugszeichen gleiche oder vergleichbare Komponenten.

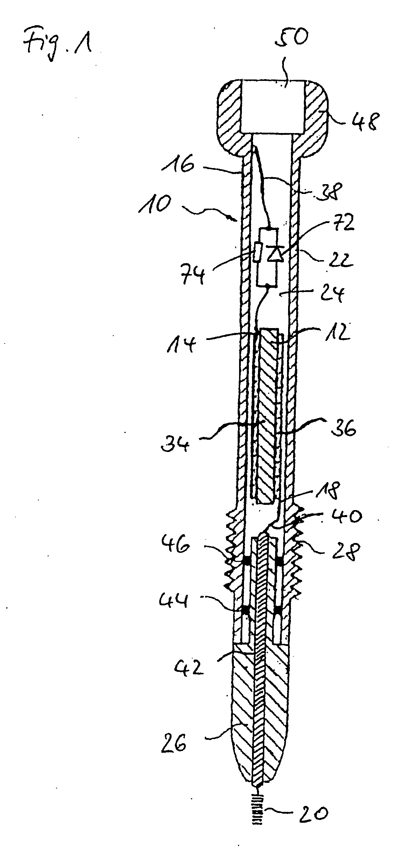

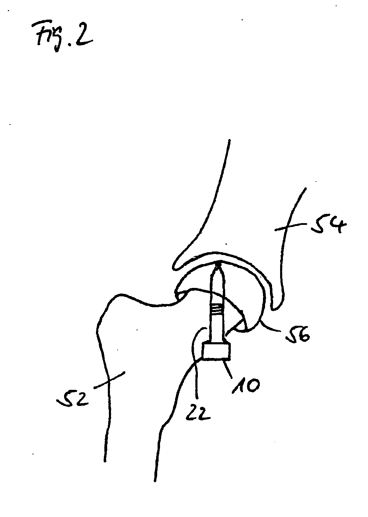

Figur 1 zeigt eine Schnittdarstellung einer erfindungsgemäßen Stimulationsvorrichtung zur Kontaktierung einer Hüftkopfkappe. Die Stimulationsvorrichtung ist als Knochenschraube 10 mit Außengewinde 28 ausgebildet. Das Außengewinde 28 ist im distalen Bereich der Knochenschraube 10 vorgesehen. Je nach Anwendung kann es ebenfalls nützlich sein, das Außengewinde im proximalen Bereich der Knochenschraube anzuordnen. In einem von dem Schaft 22 der Knochenschraube 10 umgebenen Aufnahmebereich 24 ist eine Spulenanordnung 12 vorgesehen. Die Spulenanordnung 12 umfasst einen Magnetkern 34 und eine darauf angebrachte Wicklung 36. Ein erster Pol 14 der Spulenanordnung 12 ist über eine elektrische Verbindung 38 und eine Gleichrichteranordnung 72, 74 mit dem elektrisch leitfähigen Schaft 22 der Knochenschraube 14 verbunden, die die erste Elektrode 16 bildet. Die Gleichrichteranordnung umfasst eine Diode 72 und einen zu der Diode 72 parallel geschalteten ohmschen Widerstand 74. Der zweite Pol 18 der Spulenanordnung 12 ist über eine weitere elektrische Verbindung 40 mit einem am distalen Ende der Knochenschraube 10 angeordneten elastischen Kontaktelement 20 verbunden, das die zweite Elektrode bildet. Zu diesem Zweck ist die elektrische Verbindung 40 durch ein elektrisch isolierendes Endstück 26 geführt, das beispielsweise aus Keramik oder Polyethylen besteht. Hierzu ist das Endstück 26 mit einer zentralen Bohrung 42 ausgestattet. Es sind Dichtungen 44, 46 vorgesehen, die sicherstellen, dass der Aufnahmebereich 24 der Spulenanordnung 12 gas- und flüssigkeitsdicht gegen den Außenbereich der Knochenschraube 10 abgeschlossen ist. Jegliche andere Maßnahmen zum gas- und flüssigkeitsdichten Einsetzten des Endstückes 26 in den Schaft 22 der Knochenschraube 10 ist ebenfalls denkbar. Die Knochenschraube 10 hat an ihrem proximalen Ende einen Schraubenkopf 48, der eine Öffnung 50 zum Einsetzen eines Drehwerkzeugs aufweist. Die Öffnung 50 kann beispielsweise einen Innensechskant bilden. Die durch die Diode 72 realisierte Gleichrichterschaltung kann sich vorteilhaft auf die Lokalisierung des Knochenwachstums auswirken. So wird die erste Elektrode 16 zur Anode, an der das Knochenwachstum gehemmt wird oder sogar Osteolyse stattfindet, während das Kontaktelement 20 und das hiervon kontaktierte Implantat (siehe zum Beispiel Figur 2) zur Kathode wird, so dass insbesondere in der Umgebung des Implantates das Knochenwachstum gefördert wird. Durch den zu der Diode 72 parallel geschalteten ohmschen Widerstand 74 wird eine nicht vollständige Gleichrichtung zur Verfügung gestellt. Unter Verzicht auf die genannten Vorteile der Gleichrichtung ist die Gleichrichteranordnung 72, 74 entbehrlich, so dass der erste Pol 14 der Spulenanordnung 12 direkt mit der ersten Elektrode 16 verbunden sein kann.

Figur 2 zeigt eine schematische Darstellung einer in einen Oberschenkelknochen eingebrachten Stimulationsvorrichtung. Es sind ein Oberschenkelknochen 52 und ein Beckenknochen 54 dargestellt. Auf den Oberschenkelknochen 52 ist eine Hüftkopfkappenprothese 56 aufgesetzt. Eine solche Hüftkopfkappenprothese ist häufig der Ausgangspunkt und der Herd von Bakterienkulturen, die sich unterhalb der Hüftkopfkappenprothese 56 ansiedeln. Indem die Hüftkopfkappenprothese 56 durch die Knochenschraube 10 kontaktiert wird - es ist auch der eigentlich von der Hüftkopfkappenprothese 56 verdeckte distale Bereich der Knochenschraube 10 dargestellt - wird die Hüftkopfkappenprothese 56 zur Elektrode, während der Schaft 22 der Knochenschraube 10 die Gegenelektrode bildet. Folglich wird das zwischen den Elektroden liegende Gewebe bei von außen angelegten Magnetfeldern stimuliert.

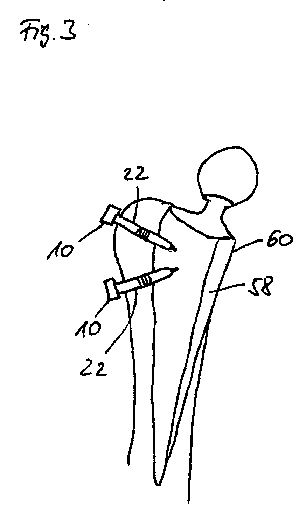

Figur 3 zeigt eine schematische Darstellung von zwei in den Oberschenkelknochen eingeschraubten Stimulationsvorrichtungen zur Kontaktierung mit dem Schaft einer Hüftgelenkprothese. Im vorliegenden Fall wird der Schaft 58 einer Hüftgelenkprothese 60 von zwei Knochenschrauben 10 der erfindungsgemäßen Art kontaktiert und auf diese Weise zur gemeinsamen Gegenelektrode zu den jeweiligen Schäften 22 der Knochenschrauben 10.

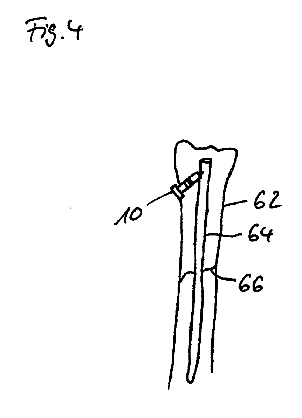

Figur 4 zeigt eine schematische Darstellung einer in einen Röhrenknochen eingebrachten Stimulationsvorrichtung zur Kontaktierung eines Marknagels. Es ist ein Röhrenknochen 62 mit einer durch einen Marknagel 64 stabilisierten Fraktur 66 dargestellt. Der Marknagel 64 wird durch eine in den Röhrenknochen 62 eingeschraubte erfindungsgemäße Knochenschraube 10 zur Elektrode.

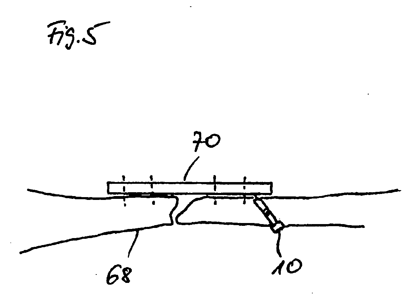

Figur 5 zeigt eine schematische Darstellung einer in einen mit einer Stützplatte stabilisierten gebrochenen Knochen. Der gebrochene Knochen 68 ist durch eine Metallplatte 70 stabilisiert. Die Verschraubungen der Metallplatte 70 sind durch unterbrochene Linien angedeutet. Indem eine erfindungsgemäße Knochenschraube 10 in den Knochen 68 eingeschraubt ist und die Metallplatte 70 kontaktiert, wird auch diese zur Elektrode.

Figur 7 zeigt einen Schnitt durch die Oberfläche einer erfindungsgemäßen Stimulationsvorrichtung. Die äußere Oberfläche der Stimulationsvorrichtung 10 ist mit einer die Oberfläche vergrößernden und die Anlagerung von Bakterien vermeidenden elektrisch leitfähigen Beschichtung, vorzugsweise aus in kolloidem Zustand vorliegenden Silberpartikeln 30, versehen. Die Beschichtung der Oberfläche wird durch eine poröse Zwischenschicht 32 vermittelt, die beispielsweise aus Kunststoff oder Keramik besteht. Ebenfalls ist möglich, dass die Silberpartikel zusätzlich oder alternativ in die Zwischenschicht eingelagert sind. Dies kann durch das Aufbringen einer Keramik-Silber-Emulsion realisiert werden. Der elektrische Kontakt zwischen der Oberfläche der Stimulationsvorrichtung 10 und der elektrisch leitfähigen Beschichtung 30 wird über Körperflüssigkeit oder durch direkten Kontakt der Oberfläche der Stimulationsvorrichtung 10 mit der Beschichtung 30 im Bereich der Poren der porösen Oberfläche zur Verfügung gestellt. Durch die bakterizide Beschichtung wird die Anlagerung von Bakterien auch ohne über die Oberfläche der Stimulationsvorrichtung zur Verfügung gestellte elektrische Potentiale behindert. Dieser Effekt wird im Rahmen der vorliegenden Erfindung durch die induzierten elektrischen Felder verstärkt. Weiterhin wird auch die Wirkung des induzierten elektrischen Feldes auf das umgebende Gewebe begünstigt, da die elektrisch leitfähige Beschichtung die Kontaktoberfläche zwischen Gewebe und Elektrode vergrößert. Im Ergebnis können hierdurch die positiven biologischen Effekte verbessert werden, oder es können unter Beibehaltung einer gegebenen Qualität einfachere und kleinere Geräte zum Einsatz kommen, was insbesondere die Spulenanordnung und die das externe magnetische Wechselfeld erzeugenden Gerätschaften betrifft.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

**Bezugszeichenliste**

| | |
|---|---|
| 10 | Knochenschraube |
| 12 | Spulenanordnung |
| 14 | erster Pol |
| 16 | Elektrode |
| 18 | zweiter Pol |
| 20 | Kontaktelement |
| 22 | Schaft |
| 24 | Aufnahmebereich |
| 26 | Endstück |
| 28 | Außengewinde |
| 30 | Beschichtung |
| 32 | Zwischenschicht |
| 34 | Magnetkern |
| 36 | Wicklung |
| 38 | elektrische Verbindung |
| 40 | elektrische Verbindung |
| 42 | Bohrung |
| 44 | Dichtung |
| 46 | Dichtung |
| 48 | Schraubenkopf |
| 50 | Öffnung |
| 52 | Oberschenkelknochen |
| 54 | Beckenknochen |
| 56 | Hüftkopfkappenprothese |
| 58 | Schaft |
| 60 | Hüftgelenkprothese |
| 62 | Röhrenknochen |
| 64 | Marknagel |
| 66 | Fraktur |
| 68 | gebrochener Knochen |
| 70 | Metallplatte |
| 72 | Diode |
| 74 | ohmscher Widerstand |

## Patentansprüche

1. Stimulationsvorrichtung (10) zur Implantation in den menschlichen Körper, mit einer Spulenanordnung (12), einer mit einem ersten Pol (14) der Spulenanordnung verbundenen ersten Elektrode (16) und einer mit einem zweiten Pol (18) der Spulenanordnung verbundenen zweiten Elektrode (20), wobei die Stimulationsvorrichtung (10) einen eine Achse definierenden Schaft (22) aufweist, die Spulenanordnung in einem radial innen liegenden Aufnahmebereich (24) des Schaftes (22) angeordnet ist und zumindest ein Teil des Schaftes (22) die erste Elektrode (16) bildet, **dadurch gekennzeichnet, dass** die zweite Elektrode (20) als elastisches Kontaktelement ausgebildet ist.

2. Stimulationsvorrichtung nach Anspruch 1, **dadurch gekennzeichnet, dass** an einem Endbereich des Schaftes (22) ein elektrisch isolierendes Endstück (26) ansetzt, durch welches eine elektrische Verbindung zu dem an der dem Schaft abgewandten Seite des Endstücks angeordneten elastischen Kontaktelement (20) geführt ist.

3. Stimulationsvorrichtung nach Anspruch 2, **dadurch gekennzeichnet, dass** das Kontaktelement (20) in dem Endstück (26) fixiert ist.

4. Stimulationsvorrichtung nach einem der vorangehenden Ansprüche, **dadurch gekennzeichnet, dass** das Kontaktelement (20) zumindest teilweise aus federhartem Stahl besteht.

5. Stimulationsvorrichtung nach einem der vorangehenden Ansprüche, **dadurch gekennzeichnet, dass** das Kontaktelement (20) zumindest teilweise aus federhartem Titan besteht.

6. Stimulationsvorrichtung nach einem der vorangehenden Ansprüche, **dadurch gekennzeichnet, dass** das Kontaktelement (20) mindestens einen gewellten Draht aufweist.

7. Stimulationsvorrichtung nach einem der vorangehenden Ansprüche, **dadurch gekennzeichnet, dass** das Kontaktelement (20) mindestens einen wendelförmigen Draht aufweist.

8. Stimulationsvorrichtung nach einem der vorangehenden Ansprüche, **dadurch gekennzeichnet, dass** die Stimulationsvorrichtung als Knochenschraube (10) ausgebildet ist, die ein Außengewinde (28) aufweist.

9. Stimulationsvorrichtung nach einem der vorangehenden Ansprüche, **dadurch gekennzeichnet, dass** die äußere Oberfläche der Stimulationsvorrichtung (10) zumindest teilweise mit einer die Oberfläche der Stimulationsvorrichtung vergrößernden und die Anlagerung von Bakterien vermeidenden, elektrisch leitfähigen Beschichtung (30) versehen ist.

10. Stimulationsvorrichtung nach Anspruch 9, **dadurch gekennzeichnet, dass** die Beschichtung (30) Silber aufweist.

11. Stimulationsvorrichtung nach Anspruch 9 oder 10, **dadurch gekennzeichnet, dass** zwischen der Oberfläche der Stimulationsvorrichtung und der Beschichtung (30) eine poröse Zwischenschicht (32) vorgesehen ist.

## Claims

1. A stimulation device (10) for the implantation into the human body comprising a coil arrangement (12), a first electrode (16) connected to a first pole (14) of the coil arrangement and a second electrode (20) connected to a second pole (18) of the coil arrangement, wherein the stimulation device (10) comprises a shaft (22) defining an axis, the coil arrangement is arranged in a radially inner accommodation area (24) of the shaft (22), and at least a part of the shaft forms the first electrode (16), **characterised in that** the second electrode (20) is formed as an elastic contact element.

2. The stimulation device according to claim 1, **characterised in that** an electrically insulating end piece (26) through which an electric connection to the elastic contact element (20) disposed on the side of the end piece opposing the shaft is lead is attached to an end section of the shaft (22).

3. The stimulation device according to claim 2, **characterised in that** the contact element (20) is fixed in the end piece (26).

4. The stimulation device according to one of the preceding claims, **characterised in that** the contact element (20), at least partly, consists of spring-hard steel.

5. The stimulation device according to one of the preceding claims, **characterised in that** the contact element (20), at least partly, consists of spring-hard titanium.

6. The stimulation device according to one of the preceding claims, **characterised in that** the contact element (20) comprises at least one undulated wire.

7. The stimulation device according to one of the preceding claims, **characterised in that** the contact element (20) comprises at least one helical wire.

8. The stimulation device according to one of the preceding claims, **characterised in that** the stimulation device is formed as a bone screw (10) comprising a male thread (28).

9. The stimulation device according to one of the preceding claims, **characterised in that** the outer surface of the stimulation device (10) is at least partly provided with an electrically conductive coating (30) enlarging the surface of the stimulation device and preventing the deposit of bacteria.

10. The stimulation device according to claim 9, **characterised in that** the coating (30) comprises silver.

11. The stimulation device according to claim 9 or 10, **characterised in that** a porous intermediate layer (32) is provided between the surface of the stimulation device and the coating (30).

## Revendications

1. Dispositif de stimulation (10) pour l'implantation dans le corps human, comportant un agencement bobine (12), une première électrode (16) connectée à un premier pôle (14) de l'agencement bobine et une deuxième électrode (20) connectée à un deuxième pôle (18) de l'agencement bobine; le dispositif de stimulation (10) comporte une hampe (22) qui définit un axe, l'agencement bobine est disposé dans une zone de réception (24) située radialement à l'intérieur et au moins une partie de la hampe (22) forme la première électrode (16), **caractérisé en ce que** la deuxième électrode (20) est un élément élastique de contact.

2. Dispositif de stimulation selon la revendication 1, **caractérisé en ce qu'**une pièce terminale (26) isolatrice électrique est raccordée à une zone terminale de la hampe (22), un raccord électrique étant mené au travers la pièce terminale à l'élément élastique de contact (20) qui est agencé sur la face de la pièce terminale détournée de la hampe.

3. Dispositif de stimulation selon la revendication 2, **caractérisé en ce qu'**un élément de contact (20) est fixé dans la pièce terminale.

4. Dispositif de stimulation selon l'une des revendications précédentes, **caractérisé en ce que** l'élément de contact (20) consiste du moins en partie d'acier qui est dur comme un ressort.

5. Dispositif de stimulation selon l'une des revendications précédentes, **caractérisé en ce que** l'élément de contact (20) consiste du moins un partie de titane qui est dur comme un ressort.

6. Dispositif de stimulation selon l'une des revendications précédentes, **caractérisé en ce que** l'élément de contact (20) comporte au moins un fil ondulé.

7. Dispositif de stimulation selon l'une des revendications précédentes, **caractérisé en ce que** l'élément de contact (20) comporte au moins un fil en spirale.

8. Dispositif de stimulation selon l'une des revendications précédentes, **caractérisé en ce que** le dispositif de stimulation est une vis d'os (10) qui comporte un filet extérieur (28).

9. Dispositif de stimulation selon l'une des revendications précédentes, **caractérisé en ce que** la surface extérieure du dispositif de stimulation (10) est garnie, du moins en partie, d'un revêtement (30) qui agrandit la surface du dispositif de stimulation et qui évite le dépôt de bactéries.

10. Dispositif de stimulation selon la revendication 9, **caractérisé en ce que** la revêtement (30) comporte de l'argent.

11. Dispositif de stimulation selon la revendication 9 ou 10, **caractérisé en ce qu'**une couche intermédiaire poreuse (32) est prévue entre la surface du dispositif de stimulation et le revêtement (30).
